# EUROPEAN PATENT SPECIFICATION

(11) **EP 1 446 015 B1**
(45) Date of publication and mention of the grant of the patent: **14.03.2018**
(21) Application number: 02801789.5
(22) Date of filing: 18.10.2002
(51) Int. Cl.: A01N 63/00, A61K 35/12, A61K 35/32, A61K 35/44, A61K 48/00

(54) **REMODELING OF TISSUES AND ORGANS**
UMMODELLIERUNG VON GEWEBEN UND ORGANEN
REMODELAGE DE TISSUS ET D'ORGANES

(30) Priority: 18.10.2001 US 347913 P; 25.07.2002 US 398448 P
(43) Date of publication of application: 18.08.2004
(73) Proprietor: LIFECELL CORPORATION, Branchburg, NJ 08876-3876 (US); Wright Medical Technology, Inc., Arlington Tennessee 38002-9501 (US)
(72) Inventor: LIVESEY, Stephen, A., Upper Black Eddy, PA 18972 (US); McQUILLAN, David, J., Doylestown, PA 18901 (US); BENIKER, Herbert, Daniel, Hillsborough, NJ 08844 (US); BOERBOOM, Lawrence, E., Bedminster, NJ 07921 (US); HAGGARD, Warren, O., Bartlett, TN 38134 (US)
(74) Representative: Lang, Johannes
(86) International application number: PCT/US2002/033456
(87) International publication number: WO 2003/032735

(56) References cited:
- EP-A1- 0 419 275
- WO-A1-00/07639
- WO-A1-99/32049
- WO-A1-99/65470
- US-A- 5 336 616
- US-A- 5 993 844
- SODIAN R. ET AL.: 'Early in vivo experience with tissue-engineered trleaflet heart valves' CIRCULATION vol. 102, no. SUPPL. III, 2000, pages II-22 - III-29, XP002959988
- PARNIGOTTO P.P. ET AL.: 'Experimental defect in rabbit urethra repaired with acellular aortic matrix' UROLOGICAL RESEARCH vol. 28, no. 1, January 2000, pages 46 - 51, XP002959989
- CHAPLIN J. ET AL.: 'Use of an acellular dermal allograft for dural replacement: an experimental study' NEUROSURGERY vol. 45, no. 2, August 1999, pages 320 - 327, XP002959990

## Description

### TECHNICAL FIELD

This invention relates to a composition for use in engineering, and more particularly to remodeling of tissues as defined in the claims.

### BACKGROUND

Due to problems inherent in transplantation of intact allogeneic or xenogeneic tissues, it is crucial that alternative strategies for replacing or repairing defective or damaged tissues be developed.

U.S. Patent Nos. 4,865,871 and 5,366,616 and copending U.S. Application Serial Nos. 09/762,174 and 10/165,790 are cited herein.

### SUMMARY

The invention is based on the observations that an acellular dermal matrix implanted into bone and cartilage defects remodeled into both tissues. In light of this finding, and the ability of a wide range of differentiated, stem cells, excluding stem cells of human embryonic origin, and progenitor cells to populate grafted matrices, it is likely that acellular matrices derived from a wide variety of collagen-based tissues will be useful in the repair of multiple defective or damaged tissues.

More specifically, the composition for use in the method provides a method of treatment. This method involves: (a) identifying a mammalian subject as having a recipient organ, or tissue, in need of repair or amelioration; and (b) placing a composition comprising a non-particulate acellular matrix made from a donor collagen-based tissue in or on the recipient organ or tissue. The recipient organ or tissue can be skin, bone, cartilage, meniscus, dermis, myocardium, periosteum, artery, vein, stomach, small intestine, large intestine, diaphragm, tendon, ligament, neural tissue, striated muscle, smooth muscle, bladder, ureter, urethra, or abdominal wall fascia. In addition, the recipient organ or tissue is different from the donor collagen-based organ or tissue. The recipient organ or tissue can be periosteum that is associated with a critical gap defect of bone. The collagen-based organ or tissue can be, for example, dermis, fascia, umbilical cord, placenta, cardiac valve, ligament, tendon, artery, vein, neural connective tissue, or ureter and the mammalian subject can be a human. The composition can also contain viable cells histocompatible with the subject, e.g. cells obtained from the mammalian subject. These cells are for example, epidermal cells, keratinocytes. endothelial cells fibroblasts, embryonic stem cells of non-human origin, adult or embryonic mesenchymal stem cells of non-human embryonic origin, umbilical cord stem cells, prochondroblasts, chondroblasts, chondrocytes, pro-osteoblasts, osteocytes, osteoclasts, monocytes, pro-cardiomyoblasts, pericytes, cardiomyoblasts, cardiomyocytes, gingival epithelial cells, or periodontal ligament stem cells. The method can further involve administration to the subject of one or more agents, e.g., a cell growth factor, an angiogenic factor, a differentiation factor, a cytokine, a hormone, and a chemokine. Such agents can be in the composition placed in or on the recipient organ or tissue or they can be injected or infused into the mammalian subject separately from the composition. Moreover the agents can be administered by administering to the subject one or more expression vectors containing one or more nucleic acid sequences encoding the one or more agents, each of the one or more nucleic acid sequences being operably linked to a transcriptional or a translational regulatory element. These expression vectors can be in one or more cells that are administered to the subject. The one or more cells can be in the composition or they can be administered to the subject separately from the composition. Embraced by the invention is another composition for use in a method of treatment. This method involves:
(a) identifying a mammalian subject as having a recipient organ, or tissue, in need of repair or amelioration; and (b) placing a composition containing a particulate acellular matrix made from a donor collagen-based organ or tissue in or on the recipient organ or tissue. The recipient organ or tissue can be skin, bone, cartilage, meniscus, dermis, myocardium, stomach, small intestine, large intestine, diaphragm, tendon, ligament, neural tissue, striated muscle, smooth muscle, bladder, or gingiva. In addition, the recipient organ or tissue is different from the donor collagen-based organ or tissue. The collagen-based organ or tissue can be, for example, dermis, fascia, umbilical cord, placenta, cardiac valve, ligament, tendon, artery, vein, neural connective tissue, or ureter and the mammalian subject can be a human. The composition can also contain viable cells histocompatible with the subject, e.g. cells obtained from the mammalian subject. These cells can be, for example, epidermal cells, keratinocytes. endothelial cells fibroblasts, embryonic stem cells of non-human embryonic origin, adult or embryonic mesenchymal stem cells of non-human embryonic origin, umbilical cord stem cells, prochondroblasts, chondroblasts, chondrocytes, pro-osteoblasts, osteocytes, osteoclasts, monocytes, pro-cardiomyoblasts, pericytes, cardiomyoblasts, cardiomyocytes, gingival epithelial cells, or periodontal ligament stem cells. The method can further involve administration to the subject of one or more agents, e.g., a cell growth factor, an angiogenic factor, a differentiation factor, a cytokine, a hormone, and a chemokine. Such agents can be in the composition placed in or on the recipient organ or tissue or they can be injected or infused into the mammalian subject separately from the composition. Moreover the agents can be administered by administering to the subject one or more expression vectors containing one or more nucleic acid sequences encoding the one or more agents, each of the one or more nucleic acid sequences being operably linked to a transcriptional or a translational regulatory element. These expression vectors can be in one or more cells that are administered to the subject. The one or more cells can be in the composition or they can be administered to the subject separately from the composition. The composition further contain demineralized bone powder. Where the recipient tissue is gingiva, the gingiva is, or is proximal to, receding gingiva. In addition, where the recipient tissue is gingiva, the gingiva can contain a dental extraction socket.

As used herein, the term "the recipient organ or tissue is different from the donor collagen-based organ or tissue" means that the recipient organ or tissue in or on which an acellular matrix is placed is different from the collagen-based organ or tissue from which that acellular matrix was made, regardless of whether the collagen-based organ or tissue was obtained from the recipient individual or from one or more other individuals. Thus, for example, where a heart valve of a host individual is the recipient tissue to be grafted with an acellular matrix, the acellular matrix is made from a tissue other than heart valve tissue, i.e., the acellular matrix cannot have been made from heart valve tissue obtained from the recipient individual or from one or more other individuals. Similarly, where skin of a host individual is the recipient tissue to be repaired with an acellular matrix, the acellular matrix is made from a tissue other than skin tissue, i.e., the acellular matrix cannot have been made from skin tissue (e.g., dermis) obtained from the recipient individual or from one or more other individuals. This concept applies to both particulate and non-particulate acellular matrices.

As used herein, the term "placing" a composition includes, without limitation, setting, injecting, infusing, pouring, packing, layering, spraying, and encasing the composition. In addition, placing "on" a recipient tissue or organ means placing in a touching relationship with the recipient tissue or organ.

As used herein, the term "operably linked" means incorporated into a genetic construct so that expression control sequences (i.e., transcriptional and translational regulatory elements) effectively control expression of a coding sequence of interest. Transcriptional and translational regulatory elements include but are not limited to inducible and non-inducible promoters, enhancers, operators and other elements that are known to those skilled in the art and that drive or otherwise regulate gene expression. Such regulatory elements include but are not limited to the cytomegalovirus hCMV immediate early gene, the early or late promoters of SV40 adenovirus, the lac system, the trp system, the TAC system, the TRC system, the major operator and promoter regions of phage A, the control regions of fd coat protein, the promoter for 3-phosphoglycerate kinase, the promoters of acid phosphatase, and the promoters of the yeast α-mating factors.

Unless otherwise defined, all technical and scientific terms used herein have the same meaning as commonly understood by one of ordinary skill in the art to which this invention pertains. In case of conflict, the present document, including definitions, will control. Preferred methods and materials are described below, although methods and materials similar or equivalent to those described herein can be used in the practice or testing of the present invention.

Other features and advantages of the invention, e.g., repairing multiple organs and tissues with acellular matrices made from collagen-based tissues, will be apparent from the following description, from the drawings and from the claims.

### DESCRIPTION OF DRAWINGS

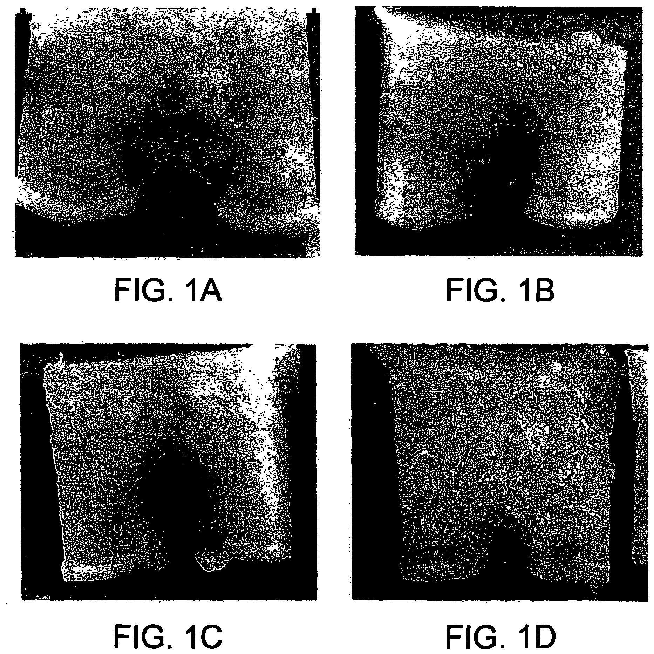

Figs. 1A-D are photographs of pig bone and cartilage tissue including lateral or medial condyles in which defects extending through the cartilage and 5mm into the subchondral bone were made. No implant was placed in a control defect (Fig. 1B). The other defects were filled with: a putty made with a high concentration (about 600mg/ml) of Cymetra® and sealed at the surface with fibrin glue (Fig. 1D); a gel made with a lower concentration (about 330mg/ml) of Cymetra combined with fibrinogen and thrombin and sealed at the surface with fibrin glue (Fig. 1C); and a paste made with a lower concentration (about 330mg/ml) of Cymetra held in place by a sheet of AlloDerm® sutured to the cartilage defect perimeter (Fig. 1A). The photographs were taken 8 weeks after surgery.

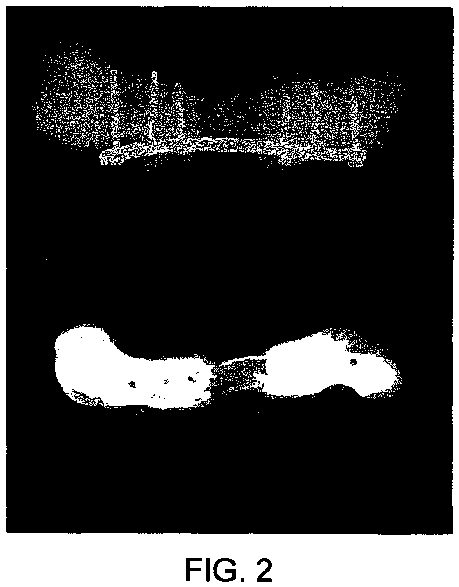

Fig. 2 is a pair of radiographs showing a critical gap defect in a pig femur that had been wrapped with a sheet of Xenoderm™ and filled with a 1:1 mixture of calcium sulfate pellets and cancellous autograft bone. The radiographs were taken 6 weeks after surgery.

### DETAILED DESCRIPTION

The experiments described in the examples indicate that implanting an acellular matrix made from a collagen-based tissue or organ in, or in direct contact with, a damaged or defective tissue or organ other than that from which the acellular matrix was made can facilitate the repair of the damaged or defective tissue or organ. As used herein, an "acellular matrix" is a matrix that: (a) is made from any of a wide range of collagen-based tissue; (b) is acellular; and (c) retains the biological and structural functions possessed by the native tissue or organ from which it was made. Biological functions retained by matrices include cell recognition and cell binding as well as the ability to support cell spreading, cell proliferation, and cell differentiation. Such functions are provided by undenatured collagenous proteins (e.g., type I collagen) and a variety of non-collagenous molecules (e.g., proteins that serve as ligands for either molecules such as integrin receptors, molecules with high charge density such glycosaminoglycans (e.g., hyaluronan) or proteoglycans, or other adhesins). Structural functions retained by useful acellular matrices include maintenance of histological architecture, maintenance of the three-dimensional array of the tissue's components and physical characteristics such as strength, elasticity, and durability, defined porosity, and retention of macromolecules. The efficiency of the biological functions of an acellular matrix can be measured, for example, by its ability to support cell proliferation and is at least 50% (e.g., at least: 50%; 60%; 70%; 80%; 90%; 95%; 98%; 99%; 99.5%; 100%; or more than 100%) of those of the native tissue or organ from which the acellular matrix is made. In addition, the integrity of the basement membrane in the acellular matrices, as measured by electron microscopy and/or immunohistochemistry, is at least 70% of that of the native tissue or organ from which the acellular matrix is made.

Thus, as indicated above, it is not necessary that the grafted matrix material be made from tissue that is identical to the surrounding host tissue but should simply be amenable to being remodeled by invading or infiltrating cells such as differentiated cells of the relevant host tissue, stem cells such as mesenchymal stem cells, or progenitor cells. Remodelling is directed by the above-described acellular matrix components and signals from the surrounding host tissue (such as cytokines, extracellular matrix components, biomechanical stimuli, and bioelectrical stimuli). The presence of mesenchymal stem cells in the bone marrow and the peripheral circulation has been documented in the literature and shown to regenerate a variety of musculoskeletal tissues [Caplan (1991) J. Orthop. Res. 9:641-650; Caplan (1994) Clin. Plast. Surg. 21:429-435; and Caplan et al. (1997) Clin Orthop. 342:254-269]. Additionally, the graft must provide some degree (greater than threshold) of tensile and biomechanical strength during the remodeling process.

It is understood that the acellular matrix can be produced from any collagen-based tissue (e.g., dermis, fascia, umbilical cords, placentae, cardiac valves, ligaments, tendons, vascular tissue (arteries and veins such as saphenous veins), neural connective tissue, or ureters), as long as the above-described properties are retained by the matrix. Moreover the tissues in which the above allografts are placed include essentially any tissue that can be remodeled by invading or infiltrating cells (see above). Relevant tissues include skeletal tissues such as bone, cartilage, ligaments, fascia, and tendon. Other tissues in which any of the above allografts can be placed include, without limitation, skin, gingiva, dura, myocardium, vascular tissue, neural tissue, striated muscle, smooth muscle, bladder wall, ureter tissue, intestine, and urethra tissue. It is understood that, for the purposes of the invention, heart muscle and skeletal muscle are not the same tissue.

Furthermore, while an acellular matrix will generally have been made from one or more individuals of the same species as the recipient of the acellular matrix graft, this is not necessarily the case. Thus, for example, an acellular matrix can have been made from a pig and be implanted in a human patient. Species that can serve as recipients of acellular matrices and donors of tissues or organs for the production of the acellular matrices include, without limitation, humans, no-human primates (e.g., monkeys, baboons, or chimpanzees), pigs, cows, horses, goats, sheep, dogs, cats, rabbits, guinea pigs, gerbils, hamsters, rats, or mice.

The form in which the acellular matrix is provided will depend on the tissue or organ from which it is derived and on the nature of the recipient tissue or organ, as well as the nature of the damage or defect in the recipient tissue or organ. Thus, for example, a matrix derived from a heart valve can be provided as a whole valve, as small sheets or strips, as pieces cut into any of a variety of shapes and/or sizes, or in a particulate form. The same concept applies to acellular matrices produced from any of the above-listed tissues and organs. It is understood that an acellular matrix useful for the invention can be made from a recipients own collagen-based tissue.

The acellular matrices can be produced by any of a variety of methods. All that is required is that the steps used in their production result in matrices with the above-described biological and structural properties. Particularly useful methods of production include those described in U.S. Patent Nos. 4,865,871 and 5,366,616 and copending U.S. Application Serial Nos. 09/762,174 and 10/165,790. Preparation of particulate acellular matrix is disclosed in WO99/65470 A1. Demineralized bone powder compositions and its use in bone repair is disclosed in EP0419275 A1. In brief, the steps involved in the production of a matrix generally include harvesting the tissue from a donor (e.g., a human cadaver or any of the above-listed mammals), chemical treatment so as to stabilize the tissue and avoid biochemical and structural degradation together with or followed by cell removal under conditions which similarly preserve biological and structural function. After thorough removal of dead and/or lysed cell components that may cause inflammation as well any bioincompatible cell-removal agents, the matrix is in principle ready for grafting and only need be processed into a desired shape or size. Alternatively, the matrix can be treated with a cryopreservation agent and cryopreserved and, optionally, freeze dried, again under conditions necessary to maintain the described biological and structural properties of the matrix. After freeze drying, the tissue can be pulverized or micronized to produce a particulate acellular matrix under similar function-preserving conditions. All steps are generally carried out under aseptic, preferably sterile, conditions.

The initial stabilizing solution arrests and prevents osmotic, hypoxic, autolytic, and proteolytic degradation, protects against microbial contamination, and reduces mechanical damage that can occur with tissues that contain, for example, smooth muscle components (e.g., blood vessels). The stabilizing solution generally contains an appropriate buffer, one or more antioxidants, one or more oncotic agents, one or more antibiotics, one or more protease inhibitors, and in some cases, a smooth muscle relaxant.

The tissue is then placed in a processing solution to remove viable cells (e.g., epithelial cells, endothelial cells, smooth muscle cells, and fibroblasts) from the structural matrix without damaging the basement membrane complex or the biological and structural integrity of the collagen matrix. The processing solution generally contains an appropriate buffer, salt, an antibiotic, one or more detergents, one or more agents to prevent cross-linking, one or more protease inhibitors, and/or one or more enzymes. Treatment of the tissue must be (a) with a processing solution containing active agents at a concentration and (b) for a time period such that degradation of the basement membrane complex is avoided and the structural integrity of the matrix is maintained.

After the tissue is decellularized, it is preferably incubated in a cryopreservation solution. This solution generally contains one or more cryoprotectants to minimize ice crystal damage to the structural matrix that could occur during freezing. If the tissue is to be freeze dried, the solution will generally also contain one or more dry-protective components, to minimize structural damage during drying and may include a combination of an organic solvent and water which undergoes neither expansion or contraction during freezing. As an alternate method, the decellularized tissue matrix can be fixed with a crosslinking agent such as glutaraldehyde and stored prior to transplantation. The cryoprotective and dry-protective agents can be the same one or more substances. If the tissue is not going to be freeze dried, it can be frozen by placing it (in a sterilized container) in a freezer at about -80°C, or by plunging it into sterile liquid nitrogen, and then storing at a temperature below -160°C until use. The sample can be thawed prior to use by, for example, immersing a sterile non-permeable vessel (see below) containing in a water bath at about 37°C or by allowing the tissue to come to room temperature under ambient conditions.

If the tissue is to be frozen and freeze dried, following incubation in the cryopreservation solution, the tissue is packaged inside a sterile vessel that is permeable to water vapor yet impermeable to bacteria, e.g., a water vapor permeable pouch or glass vial. One side of a preferred pouch consists of medical grade porous Tyvek® membrane, a trademarked product of DuPont Company of Wilmington, DE. This membrane is porous to water vapor and impervious to bacteria and dust. The Tyvek membrane is heat sealed to a impermeable polythylene laminate sheet, leaving one side open, thus forming a two-sided pouch. The open pouch is sterilized by irradiation (e.g., gamma irradiation) prior to use. The tissue is aseptically placed (through the open side) into the sterile pouch. The open side is then aseptically heat sealed to close the pouch. The packaged tissue is henceforth protected from microbial contamination throughout subsequent processing steps.

The vessel containing the tissue is cooled to a low temperature at a specified rate which is compatible with the specific cryoprotectant to minimize the development of damaging hexagonal ice and to generate the less stable ice forms of amorphous and cubic phases. See U.S. Patent No. 5,336,616 for examples of appropriate cooling protocols. The tissue is then dried at a low temperature under vacuum conditions, such that water vapor is removed sequentially from each ice crystal phase without ice recrystallization. Such drying is achieved either by conventional freeze drying or by using a previously patented molecular distillation dryer. Suitable molecular distillation dryers can be obtained from LifeCell Corporation in the Woodlands, TX and are described in U.S. Pat. Nos. 4,567,847 and 4,799,361. At the completion of the drying of the samples in the water vapor permeable vessel, the vacuum of the freeze drying apparatus is reversed with a dry inert gas such as nitrogen, helium or argon. While being maintained in the same gaseous environment, the semipermeable vessel is placed inside an impervious (i.e., impermeable to water vapor as well as mocroorganims) vessel (e.g., a pouch) which is further sealed, e.g., by heat and/or pressure. Where the tissue sample was frozen and dried in a glass vial, the vial is sealed under vacuum with an appropriate inert stopper and the vacuum of the drying apparatus reversed with an inert gas prior to unloading. In either case, the final product is hermetically sealed in an inert gaseous atmosphere.

The freeze dried tissue may be stored under these conditions for extended time periods under ambient refrigerated conditions. Transportation may be accomplished via standard carriers and under standard conditions relative to normal temperature exposure and delivery times.

Generally (but not necessarily) the dried tissue is rehydrated prior to transplantation. It is important to minimize osmotic forces and surface tension effects during rehydration. The aim in rehydration is to augment the selective preservation of the extracellular support matrix. Appropriate rehydration may be accomplished by, for example, an initial incubation of the dried tissue in an environment of about 100% relative humidity, followed by immersion in a suitable rehydration solution. Alternatively, the dried tissue may be directly immersed in the rehydration solution without prior incubation, in a high humidity environment. Rehydration should not cause osmotic damage to the sample. Vapor rehydration should ideally achieve a residual moisture level of at least 15% and fluid rehydration should result in a tissue moisture level of between 20% and 70%. Depending on the tissue to be rehydrated, the rehydration solution can be, for example, normal saline, Ringer's lactate, or a standard cell culture medium. Where the tissue is subject to the action of endogenous collagenases, elastases or residual autolytic activity from previously removed cells, additives to the rehydration solution are made and include protease inhibitors. Where residual free radical activity is present, agents to protect against free radicals are used including antioxidants, and enzymatic agents that protect against free radical damage. Antibiotics may also be included to inhibit bacterial contamination. Oncotic agents being in the form of proteoglycans, dextran and/or amino acids may also be included to prevent osmotic damage to the matrix during rehydration. Rehydration of a dry sample is especially suited to this process as it allows rapid and uniform distribution of the components of the rehydration solution. In addition, the rehydration solutions may contain specific components not used previously, for example, diphosphonates to inhibit alkaline phosphatase and prevent subsequent calcification. Agents may also be included in the rehydration solution to stimulate neovascularization and host cell infiltration following transplantation of the rehydrated extracellular matrix. Alternatively, rehydration may be performed in a solution containing a cross-linking agent such as glutaraldehyde.

Histocompatible, viable cells can be restored to the acellular matrices to produce a permanently accepted graft that may be remodeled by the host. This is generally done just prior to after placing of the acellular matrix in a mammalian subject. Where the matrix has been freeze dried, it will be done after rehydration. In a preferred embodiment, histocompatible viable cells may be added to the matrices by standard in vitro cell coculturing techniques prior to transplantation, or by in vivo repopulation following transplantation.

The cell types used for reconstitution will depend on the nature of the tissue or organ to which the acellular matrix is being remodelled. For example, the primary requirement for reconstitution of full-thickness skin with an acellular matrix is the restoration of epidermal cells or keratinocytes. The cells may be derived from the intended recipient patient, in the form of a small meshed split-skin graft or as isolated keratinocytes expanded to sheets under cell culture conditions or as keratinocyte stem cells applied to the acellular matrix. Alternatively, allogeneic keratinocytes derived from foreskin or fetal origin, may be used to culture and reconstitute the epidermis.

The most important cell for reconstitution of heart valves and vascular conduits is the endothelial cell, which lines the inner surface of the tissue. Endothelial cells may also be expanded in culture, and may be derived directly from the intended recipient patient or from umbilical arteries or veins.

Other cells with which the matrices can be repopulated include, but are not limited to, firbroblasts, embryonic stem cells (ESC) of non-human embryonic origin, adult or embryonic mesenchymal stem cells (MSC) of non-human origin, prochondroblasts, chondroblasts, chondrocytes, pro-osteoblasts, osteocytes, osteoclasts, monocytes, pro-cardiomyoblasts, pericytes, cardiomyoblasts, cardiomyocytes, gingival epithelial cells, or periodontal ligament stem cells. Naturally, the acellular matrices can be repopulated with combinations of two more (e.g., two, three, four, five, six, seven, eight, nine, or ten) of these cell- types.

Following removal of cells, following freezing, following drying, following drying and rehydration, or following reconstitution of the acellular matrix (whether or not frozen or dried) with appropriate cells, the acellular matrix can be transported to the appropriate hospital or treatment facility. The choice of the final composition of the product will be dependent on the specific intended clinical application.

Reagents and methods for carrying out all the above steps are known in the art. Suitable reagents and methods are described in, for example, U.S. Patent No 5,336,616.

Particulate acellular matrices can be made from any of the above described non-particulate acellular matrices by any process that results in the preservation of the biological and structural functions described above and, in particular, damage to collagen fibers, including sheared fiber ends, should be minimized. Many known wet and drying processes for making particulate matrices do not so preserve the structural integrity of collagen fibers.

One appropriate method is described in U.S. Patent Application Serial No.09/762,174. The process is briefly described below with respect to a freeze dried dermal acellular matrix but one of skill in the art could readily adapt the method for use with freeze dried acellular matrices derived from any of the other tissues listed herein.

The acellular dermal matrix can be cut into strips (using, for example, a Zimmer mesher fitted with a non-interrupting "continuous" cutting wheel). The resulting long strips are then cut into lengths of about 1cm to about 2cm. A homogenizer and sterilized homogenizer probe (e.g., a LabTeck Macro homogenizer available from OMNI International, Warrenton, VA) is assembled and cooled to cryogenic temperatures (i.e., about -196°C to about -160°C) using sterile liquid nitrogen which is poured into the homogenizer tower. Once the homogenizer has reached a cryogenic temperature, cut pieces of acellular matrix are added to the homogenizing tower containing the liquid nitrogen. The homogenizer is then activated so as to cryogenically fracture the pieces of acellular matrix. The time and duration of the cryogenic fracturing step will depend upon the homogenizer utilized, the size of the homogenizing chamber, and the speed and time at which the homogenizer is operated, and are readily determinable by one skilled in the art. As an alternative, the cryofracturing process can be conducted in cryomill cooled to a cryogenic temperature.

The cryofractured particulate acellular tissue matrix is, optionally, sorted by particle size by washing the product of the homogenization with sterile liquid nitrogen through a series of metal screens that have also been cooled to a cryogenic temperature. It is generally useful to eliminate large undesired particles with a screen with a relatively large pore size before proceeding to one (or more screens) with a smaller pore size. Once isolated, the particles can be freeze dried to ensure that any residual moisture that may have been absorbed during the procedure is removed. The final product is a powder (usually white or off-white) generally having a particle size of about 1 micron to about 900 microns, about 30 microns to about 750 microns, or about 150 to about 300 microns. The material is readily rehydrated by suspension in normal saline or any other suitable rehydrating agent known in the art. It may also be suspended in any suitable carriers known in the art (see, for example, U.S. Patent No. 5,284,655. If suspended at a high concentration (e.g., at about 600mg/ml), the particulate acellular matrices can form a "putty", and if suspended at a somewhat lower concentration (e.g., about 330 mg/ml), it can form a "paste". Such putties and pastes can conveniently be packed into, for example, holes, gaps, or spaces of any shape in tissues and organs so as to substantially fill such holes, gaps, or spaces.

One highly suitable freeze dried acellular matrix is produced from human dermis by the LifeCell Corporation (Branchburg, NJ) and marketed in the form of small sheets as AlloDerm®. Such sheets are market by the LifeCell Corporation as rectangular sheets with the dimensions of, for example, 1cm x 2cm, 3cm x 7cm, 4cm x 8cm, and 5cm x 10cm. The cryoprotectant used for freezing and drying Alloderm is a solution of 35% maltodextrin and 10mM ethylenediaminetetraacetate (EDTA). Thus, the final dried product contains about 60% by weight acellular matrix and about 40% by weight maltodextrin. The LifeCell Corporation also makes an analogous product made from pig dermis as XenoDerm™ having the same proportions of acellular matrix and maltodextrin as AlloDerm. In addition, the LifeCell Corporation markets a particulate acellular dermal matrix made by cryofracturing AlloDerm (as described above) under the name Cymetra®. The particle size for Cymetra is in the range of about 60 microns to about 150 microns as determined by mass.

The form of acellular matrix used in any particular instance will depend on the tissue or organ to which it is to be applied. Generally non-particulate acellular matrices that are provided in dry form (e.g., AlloDerm) are rehydrated in a sterile physiological solution (e.g., saline) before use. However they can also be used dry.

Sheets of acellular matrix (optionally cut to an appropriate size) can be: (a) wrapped around a tissue or organ that is damaged or that contains a defect; (b) placed on the surface of a tissue or organ that is damaged or has a defect; or (c) rolled up and inserted into a cavity, gap, or space in the tissue or organ. Such cavities, gaps, or spaces can be, for example: (i) of traumatic origin, (ii) due to removal of diseased tissue (e.g., infarcted myocardial tissue), or (iii) due to removal of malignant or non-malignant tumors. The acellular matrices can be used to augment or ameliorate underdeveloped tissues or organs or to augment or reconfigure deformed tissues or organs. One or more (e.g., one, two, three, four, five, six, seven, eight, nine, ten, 12, 14, 16, 18, 20, 25, 30, or more) such strips can be used at any particular site. The grafts can be held in place by, for example, sutures, staples, tacks, or tissue glues or sealants known in the art. Alternatively, if, for example, packed sufficiently tightly into a defect or cavity, they may need no securing device. Particulate acellular matrices can be suspended in a sterile pharmaceutically acceptable carrier (e.g., normal saline) and injected via hypodermic needle into a site of interest. Alternatively, the dry powdered matrix or a suspension can be sprayed onto into or onto a site or interest. A suspension can be also be poured into or onto particular site. In addition, by mixing the particulate acellular matrix with a relatively small amount of liquid carrier, a "putty" can be made. Such a putty, or even dry particulate acellular matrix, can be layered, packed, or encased in any of the gaps, cavities, or spaces in organs or tissues mentioned above. Moreover, a non-particulate acellular matrix can be used in combination with particulate acellular matrix. For example, a cavity in bone could be packed with a putty (as described above) and covered with a sheet of acellular matrix.

It is understood that an acellular matrix can be applied to a tissue or organ in order to repair or regenerate that tissue or organ and/or a neighboring tissue or organ. Thus, for example, a strip of acellular matrix can be wrapped around a critical gap defect of a long bone to generate a perisoteum equivalent surrounding the gap defect and the periosteum equivalent can in turn stimulate the production of bone within the gap in the bone. Similarly, by implanting an acellular matrix in an dental extraction socket, injured gum tissue can be repaired and/or replaced and the "new" gum tissue can assist in the repair and/or regeneration of any bone in the base of the socket that may have been lost as a result, for example, of tooth extraction. In regard to gum tissue (gingiva), receding gums can also be replaced by injection of a suspension, or by packing of a putty of particulate acellular matrix into the appropriate gum tissue. Again, in addition to repairing the gingival tissue, this treatment can result in regeneration of bone lost as a result of periodontal disease and/or tooth extraction. Compositions used to treat any of the above gingival defects can contain one or more other components listed herein, e.g., demineralized bone powder, growth factors, or stem cells.

Both non-particulate and particulate acellular matrices can be used in combination with other scaffold or physical support components. For example, one or more sheets of acellular matrix can be layered with one or more sheets made from a biological material other than acellular matrix, e.g., irradiated cartilage supplied by a tissue bank such as LifeNet, Virginia Beach, VA, or bone wedges and shapes supplied by, for example, the Osteotech Corporation, Edentown, NJ. Alternatively, such non-acellular matrix sheets can be made from synthetic materials, e.g., polyglycolic acid or hydrogels such that supplied by Biocure, Inc., Atlanta, GA. Other suitable scaffold or physical support materials are disclosed in U.S. Patent No. 5,885,829. It is understood that such additional scaffold or physical support components can be in any convenient size or shape, e.g., sheets, cubes, rectangles, discs, spheres, or particles (as described above for particulate acellular matrices).

Other active substances that can be mixed with particulate acellular matrices or impregnated into non-particulate acellular matrices include bone powder, demineralized bone powder, and any of those disclosed above.

Factors that can be incorporated into the matrices, administered to the placement site of an acellular matrix graft, or administered systemically include any of a wide range of cell growth factors, angiogenic factors, differentiation factors, cytokines, hormones, and chemokines known in the art. Any combination of two or more of the factors can be administered to a subject by any of the means recited below. Examples of relevant factors include fibroblast growth factors (FGF) (e.g., FGF1-10), epidermal growth factor, keratinocyte growth factor, vascular endothelial cell growth factors (VEGF) (e.g., VEGF A, B, C, D, and E), platelet-derived growth factor (PDGF), interferons (IFN) (e.g., IFN-α, β, or γ), transforming growth factors (TGF) (e.g., TGFα or β), tumor necrosis factor-α, an interleukin (IL) (e.g., IL-1 - IL-18), Osterix, Hedgehogs (e.g., sonic or desert), SOX9, bone morphogenic proteins, parathyroid hormone, calcitonin prostaglandins, or ascorbic acid.

Factors that are proteins can also be delivered to a recipient subject by administering to the subject: (a) expression vectors (e.g., plasmids or viral vectors) containing nucleic acid sequences encoding any one or more of the above factors that are proteins; or (b) cells that have been transfected or transduced (stably or transiently) with such expression vectors. Such transfected or transduced cells will preferably be derived from, or histocompatible with, the recipient. However, it is possible that only short exposure to the factor is required and thus histo-incompatible cells can also be used. The cells can be incorporated into the acellular matrices (particulate or non-particulate) prior to the matrices being placed in the subject. Alternatively, they can be injected into an acellular matrix already in place in a subject, into a region close to an acellular matrix already in place in a subject, or systemically. Naturally, administration of the acellular matrices and/or any of the other substances or factors mentioned above can be single, or multiple (e.g., two, three, four, five, six, seven, eight, nine, 10, 15, 20, 25, 30, 35, 40, 50, 60, 80, 90, 100, or as many as needed). Where multiple, the administrations can be at time intervals readily determinable by one skilled in art. Doses of the various substances and factors will vary greatly according to the species, age, weight, size, and sex of the subject and are also readily determinable by a skilled artisan.

Conditions for which the matrices can be used are multiple. Thus, for example, they can be used for the repair of bones and/or cartilage with any of the above-described damage or defects. Both particulate and non-particulate acellular matrices can be used in any of the forms and by any of the processes listed above. Bones to which such a composition for use in of treatment can be applied include, without limitation, long bones (e.g., tibia, femur, humerus, radius, ulna, or fibula), bones of the hand and foot (e.g., calcaneas bone or scaphoid bone), bones of the head and neck (e.g., temporal bone, parietal bone, frontal bone, maxilla, mandible), or vertebrae. As mentioned above, critical gap defects of bone can be treated with acellular matrices. In such critical gap defects, the gaps can be filled with, example, a putty of particulate acellular matrix or packed sheets of acellular matrix and wrapped with sheets of acellular matrix. Alternatively, the gaps can be wrapped with a sheet of acellular matrix and filled with other materials (see below). In all these bone and/or cartilage treatments, additional materials can be used to further assist in the repair process. For example, the gap can be filled cancellous bone and or calcium sulfate pellets and particulate acellular matrices can be delivered to sites of bone damage or bone defects mixed with demineralized bone powder. In addition, acellular matrices can be combined with bone marrow and/or bone chips from the recipient.

Acellular matrices can also be used to repair fascia, e.g., abdominal wall fascia or pelvic floor fascia. In such methods, strips of acellular matrix are generally attached to the abdominal or pelvic floor by, for example, suturing either to the surrounding fascia or host tissue or to stable ligaments or tendons such as Cooper's ligament.

Infarcted myocardium is another candidate for remodeling repair by acellular matrices. Contrary to prior dogma, it is now known that not all cardiac myocytes have lost proliferative and thus regenerative potential [e.g., Beltrami et al. (2001) New. Engl. J. Med. 344:1750-1757; Kajstura et al. (1998) Proc. Nat'l. Acad. Sci. USA 95:8801-8805]. Moreover, stem cells, present for example in bone marrow and blood and as pericytes associated with blood vessels, can differentiate to cardiac myocytes. Either the infarcted tissue itself can be removed and replaced with a sheet of acellular matrix cut to an appropriate size or a suspension of particulate acellular matrix can be injected into the infarcted tissue. Congenital heart hypoplasia, or other structural defects, can be repaired by, for example, making an incision in the tissue, expanding the gap created by the incision, and inserting a sheet of acellular matrix cut to the desired size, or placing sheets of acellular matrix on the epicardial and endocardial surfaces and placing particulate acellular matrix between them.. It is understood that, in certain conditions, creating a gap by incision may not be sufficient and it may be necessary to excise some tissue. Naturally, one of skill in the art will appreciate that the acellular matrices can be used similarly to repair damage to or defects in other types of muscle, e.g., ureter or bladder or skeletal muscle such as biceps, pectoralis, or latissimus.

Moreover, sheets of acellular matrix can be used to repair or replace damaged or removed intestinal tissue, including the esophagus, stomach and small and large intestines. In this case, the sheets of acellular matrix can be used to repair perforations or holes in the intestine. Alternatively, a sheet of acellular matrix can be formed, for example, into a cylinder which can be used to fill a gap in the intestine (e.g., a gap created by surgery to remove a tumor or a diseased segment of intestine). Such methods can be used to treat, for example, diaphragmatic hernias. It will be understood that an acellular matrix in sheet form can also be used to repair the diaphragm itself in this condition as well as in other conditions of the diaphragm requiring repair or replacement, or addition of tissue.

The following examples serve to illustrate the invention.

### EXAMPLES

### Example 1. Remodeling of an Acellular Dermal Matrix to Bone and Cartilage Assessed Seven Days After Creation of Full-thickness Osteochondral Defects

In this first example, reparative processes at an early time post-implant (1 week) were examined to demonstrate early remodeling events, including repopulation, revascularization, and integration. In addition, different configurations of acellular matrix materials were tested. A Yucatan minipig model was used to assess the efficacy of Xenoderm (acellular porcine dermal matrix sheet) and micronized particulate Xenoderm (cryofractured acellular porcine dermal matrix) to repair articular cartilage and bone defects. Animal husbandry and surgery were performed in accordance with the Institutional Animal Care and Use Committee (IACUC) requirements. In general, animals were anesthetized with Telazol (8 mg/Kg), Ketamine (4 mg/Kg) and Xylazine (4 mg/Kg), intramuscularly (IM). They were entubated and maintained on 2-3% Isoflurane and 1-2 L of O₂/minute. Pre-operative medications included approximately 40 mg/Kg Cefazolin intravenously (IV), 0.007 mg/Kg Buprenorphine IM, and 0.01 mg/Kg Glycopyrrolate IM. The post-operative antimicrobial agent was 3.0 to 3.5 g Cefazolin IV. Post-operative analgesia included 0.007 mg/Kg Buprenorphine IM and 50, and 75µg/hour Fentanyl (transdermal) patches placed every 1-3 days as needed.

### Animal 1 (ID# 80-6). Cartilage repair model.

Only the rear right leg of the animal was operated on as there was a concern that exposure and surgery to the stifle joint (knee joint) of both legs would result in excessive lameness and consequent pain and suffering.

A lateral incision was made extending from the distal femur to the proximal tibia exposing the joint capsule. An incision was made into the joint space exposing lateral and medial condyles. A 6 mm drill bit with a sleeve to prevent over drilling of the defect depth was used to create the final defect. Sterile saline was used to hydrate test matrices prior to implantation. After irrigation of the defect with saline to remove bone debris and spilled marrow elements, the appropriate matrix compound was packed into the defect site. The joint space was flushed with saline and closed with 3-0 PDS (polydioxanosulfate suture, Ethicon Inc, Sommerville NJ) in a discontinuous suture pattern. The muscle and subcutaneous layers were closed with 2-0 Prolene (polypropylene suture, Ethicon Inc., Sommerville NJ) in a continuous suture pattern.

A full-thickness defect 6 mm in diameter and extending 6-8 mm into the subchondral bone was created in the lateral condyle. The defect was filled with micronized XenoDerm (porcine equivalent of Cymetra) resuspended at about 330 mg/ml in sterile saline. A sheet (2 cm²) of XenoDerm (porcine equivalent of AlloDerm) was cut to size, placed over the defect and fixed in place by use of Poly L-Lactide (PLLA) bioabsorbable tacks (AutoTac System, BioHorizons, Birmingham AL) in non-weight bearing points of the condyle. After positioning of the XenoDerm sheet, filling of the defect was ensured by injection of further micronized Xenoderm suspension through the sheet using a 26-guage needle.

An identical defect (6-mm diameter and 6-8 mm penetration of subchondral bone) was created in the medial condyle. The defect was filled with a 6 mm wide strip of XenoDerm in a "cigar roll" configuration. After implantation of the "cigar roll" strip, the space remaining above the implant was filled with three circular 6 mm discs of XenoDerm sheet press-fitted into the defect. A sheet (2 cm²) of XenoDerm was cut to size, placed over the condyle, and fixed in place with seven equally spaced sutures using 6-0 PDS.

### Animal 2 (ID# 80-2). Bone graft model

Both rear legs were operated on as it was considered that the surgery would be less traumatic compared to accessing the joint space. A lateral approach to the stifle joint (knee joint) was used with a skin incision extending from the distal aspect of the femur to the tibial tuberosity. The subcutaneous tissue was dissected and a periosteal elevator used to clear fascial attachments to the distal femur and proximal tibia.

Defects of 1 cm diameter, penetrating 4-5 mm into the bone were created on the lateral aspects of the distal femur and proximal tibia of both rear legs, using a 1 cm diameter drill bit.

The defect on the distal femur of the right leg was filled with pre-cut 1-cm diameter XenoDerm sheets. A 2-cm² sheet of XenoDerm was sutured using 6-0 Prolene to the surrounding periosteum covering the implant. The defect in the proximal tibia of the right leg was filled with micronized XenoDerm, rehydrated in sterile saline to about 330 mg/ml. The implant was held in place by the close apposition of overlying fascia and muscle at closing of the wound.

The defect on the distal femur of the left leg was filled with dry micronized XenoDerm. A 2-cm² sheet of XenoDerm was placed over the defect and fixed in place by 4 PLLA tacks (AutoTac, Biohorizons, Birmingham AL). The defect in the proximal tibia of the left leg was filled with autologous bone that was obtained from pooling the bone harvested during creation of the four bone defects. The autologous bone was maintained moist with sterile saline and morcelized with a mortar and pestle prior to implant. The implant was held in place by the close apposition of overlying fascia and muscle at closing of the wound.

Animals were placed in a sling for at least 2 hours following surgery. Following recovery from anesthesia, animals were maintained in restricted pens that allowed restricted movement and weight-bearing. Twenty four hours following surgery, both animals were mobile. Animal 1 (cartilage defect) was favoring the surgical leg but was doing limited weight-bearing on the operated limb. Animal 2 (bone defects) was mobile.

Seven days after surgery, the animals were sacrificed and the rear limbs of both animals disarticulated at the hip joint and the bone implant limbs of animal 2 were taken for x-ray. The joint regions were dissected from the limbs and subjected to gross and microscopic examination.

With respect to animal 1 (#80-6), the following gross observations were made.

### (a) Lateral femoral condyle

In the micronized XenoDerm filled defect, the XenoDerm sheet had become free but was held in place at one tack point. Hemorrhage was apparent in the joint at the interface between the condyles and patellar articulating surface. The defect was slightly concave (1-1.5 mm below surface), reddish, and bloody in appearance. The tack holes were clearly visible and black in color. The cartilage-bone block was excised and placed in 10% formalin fixative for 4 days at 4°C. The XenoDerm sheet was separately fixed in 10% formalin under the same conditions.

### (b) Medial femoral condyle

The XenoDerm flap was intact and well fixed to the cartilage surface. The sutures were cut and the flap placed in 10% formalin, as above. The suture thread was adherent to the defect. The defect was continuous with the cartilage surface and firm to touch and there was some blood. The overall cartilage surface looked clear. The cartilage-bone block was excised and fixed in 10% formalin as described above.

Both blocks were removed from formalin after 4 days, and bisected with a razor blade to 3-4 mm thickness for processing in "decalcification" solvent.

In summary, the gross observations made it clear that 1 week following implantation the implant materials were present in the osteochondral defects, that there was continuity with surrounding tissue, and that there was retention of volume.

With respect to animal 2 (#80-2), the following gross observations were made.

### Gross analysis

### (a) Right leg, distal femur

The XenoDerm sheet covering the defect was in place, although significant hematoma around the surgical site was evident. There was a slight depression at the center of the flap (about 1 mm). The entire implant and surrounding bone was excised using a bone saw and the block placed in 10% formalin. After 4 days in formalin, the block was bisected and the gross appearance of the implant observed.

### (b) Right leg, proximal tibia

The margins of the defect were not easily distinguished, and significant hematoma was evident. The surface of the implant was irregular, yet firm to penetration with a probe. The entire implant and surrounding bone was excised using a bone saw and the block placed in 10% formalin. After 4 days in formalin, the block was bisected and the gross appearance of the implant observed.

### (c) Left leg, distal femur

The XenoDerm sheet fixed with PLLA tacks was intact and unremarkable, and appeared to be adherent to the surrounding periosteum. The implant material underlying the sheet was firm to probing. The entire implant and surrounding bone was excised using a bone saw and the block placed in 10% formalin. After 4 days in formalin, the block was bisected and the gross appearance of the implant observed.

### (d) Left leg, proximal tibia

The autologous bone implant exhibited a rough surface with protruding bony fragments. The implant was resistant to probing. The entire implant and surrounding bone was excised using a bone saw and the block placed in 10% formalin. After 4 days in formalin, the block was bisected and the gross appearance of the implant observed.

At a gross level, all bone implants exhibited retention of volume and good contact with surrounding tissue. There was no evidence of infection or detectable rejection of the implant.

### Histological Analysis

Effective tissue repair and regeneration requires that the implant material be revascularized. Revascularization facilitates repopulation by reparative cells that drive the remodeling process. The histological analysis of the osteochondral defect created in animal 1, filled with micronized XenoDerm, is representative of these processes occurring in all acellular implant configurations. Hemotoxylin and eosin (H&E) staining of the osteochondral defect 7 days post-implant indicated that the approximate dimensions of the original defect are clearly defined, with a 6-mm diameter hole penetrating well into the underlying subchondral bone. The surrounding host articular cartilage, and underlying trabecular bone and bone marrow elements were also identified. There was evidence, even at this early time point, of in-growth of cartilage at the surface, and new bone formation along both the walls and base of the defect. Effective integration between the implant and surrounding host cartilage, extensive revascularization as evidenced by numerous blood vessels throughout the implant, and trabecular extensions, representative of new bone formation, arising from the base of the implant were observed. These phenomena were seen in all acellular implant material to varying degrees, depending on the implant configuration. The micronized XenoDerm exhibited a greater degreee of revascularization and cellular repopulation compared with the sheet XenoDerm at this early time point. However, the general conclusion was that, at 7 days, appropriate remodeling events were occurring that would facilitate cartilage and bone repair.

### Example 2. Remodeling of an Acellular Dermal Matrix to Bone and Cartilage Assessed Eight Weeks After Creation of Full-thickness Osteochondral Defects

A study using the Yucatan minipig osteochondral plug defect model was conducted to demonstrate the efficacy of acellular dermal matrix scaffolds for repairing boney defects underlying articular cartilage defects. Three formulations of implants were evaluated and compared to a defect not filled with any formulation. The formulations tested were: (1) micronized XenoDerm putty (∼600 mg/ml) sealed at the surface with fibrin glue (2) micronized XenoDerm (∼330 mg/ml) combined with fibrinogen and thrombin to create a gel sealed at the surface with fibrin glue, or (3) micronized XenoDerm paste (∼330 mg/ml) held in place by a sheet of AlloDerm sutured to the cartilage defect perimeter. Thus the acellular matrix components of formulations (1) and (3) differed only with respect to the concentration of micronized (particulate) XenoDerm.

Full-thickness defects 6.4 mm in diameter and extending through the cartilage and 5 mm into the subchondral bone were created unilaterally on the medial and femoral condyles of 2 skeletally-mature Yucatan minipigs. Skeletally-mature Yucatan minipigs were chosen because of their anatomical size and cartilage thickness approximating that of humans. The 2 animals chosen were of identical age and similar weight (82 kg and 84 kg), and were radiographically screened pre-operatively to ensure proper size, skeletal maturity, and that no obvious osseous abnormalities existed.

Both pigs were anesthetized with Telazol (8 mg/Kg), Ketamine (4 mg/Kg), and Xylazine (4 mg/Kg), IM. They were entubated and maintained on 2-3% Isoflurane and 1-2 L of O₂/minute. Pre-operative medications included approximately 40 mg/Kg Cefazolin intravenously IV, 0.007 mg/Kg Buprenorphine IM, and 0.01 mg/Kg Glycopyrrolate IM. The post-operative antimicrobial was 3.0 to 3.5 g Cefazolin IV. Post-operative analgesia included 0.007 mg/Kg Buprenorphine IM and 50, and 75µg/hour Fentanyl (transdermal) patches placed every 1-3 days as needed.

A lateral incision was made extending from the distal femur to the proximal tibia exposing the joint capsule. An incision was made into the joint space exposing lateral and medial condyles. A 6.4 mm drill bit with a sleeve to prevent over drilling of the defect depth (5 mm) was used to create the final defect. Sterile saline was used to hydrate test compounds prior to implantation. After irrigation of the defect with saline to remove bone debris and spilled marrow elements, the appropriate compound was packed into the defect site with a syringe and blunt probe. Sufficient material was placed into the defect so that it was flush with the articulating surface. The joint space was flushed with saline and closed with 3-0 PDS in a simple interrupted suture pattern. The muscle and subcutaneous layers were closed with 2-0 Prolene in a continuous suture pattern.

Animals were placed in a pig sling for at least 2 hours after the end of surgery. A Robert-Jones bandage was applied to the operated leg to decrease excess motion at the stifle (knee) joint. Animals were euthanized 8 weeks after surgery, hind limbs removed and processed for analysis.

Bone and cartilage healing was evaluated grossly and histologically using routine protocols. Joints were exposed and defects photographed. Defects were excised en bloc and placed in formalin fixative for 3 days. After initial fixation, defects were dissected into 2 halves to visualize remodeling through the depth of the osteochondral defect. One half was subjected to limited de-calcification, sectioned, and stained with H&E and other stains as required. The second half was processed for immunocytochemistry for collagen type II expression.

### Gross Analysis

Each harvested defect was inspected for gross appearance. This subjective analysis apportions points based on the formation of intra-articular lesions, restoration of articular surface, erosion and appearance of the cartilage. The gross grading scale is set forth in the following:

| | Grade |
|---|---|
| Intra-articular adhesions | |
| None = | 2 |
| Minimal/fine loose fibrous tissue = | 1 |
| Major/dense fibrous tissue = | 0 |
| | |

| Restoration of articular cartilage | |
|---|---|
| Complete = | 2 |
| Partial = | 1 |
| None = | 0 |
| | |

| Erosion of cartilage | |
|---|---|
| None = | 2 |
| Defect site and border = | 1 |
| Defect site and adjacent normal cartilage = | 0 |
| | |

| Appearance of cartilage | |
|---|---|
| Translucent = | 2 |
| Opaque = | 1 |
| Discolored or irregular = | 0 |
| | |
| TOTAL POSSIBLE SCORE= | 8 |

The gross scoring for the 3 implant materials and the empty control defect were as follows:

| Implant material | Total Score |
|---|---|
| Empty defect | 3 |
| Cymetra | 5 |
| Cymetra + Fibrin | 6 |
| Cymetra Putty | 4 |

These gross observations indicate a marked improved repair of the cartilage surface for the defect filled with Cymetra/Fibrin compared with the no treatment control.

Fixed blocks were bisected and photographed, and are shown in Fig. 1. This analysis shows the repair at 8 weeks in the underlying trabecular bone. Significant bone repair is evident in the defect filled with Cymetra Putty (Fig. 1, panel D) compared with the empty defect (Fig. 1, panel B). The combination of a fibrin polymer with Cymetra appears to have inhibited bone remodeling (Fig. 1, panel C), and the defect filled with Cymetra paste (Fig.1, panel A) alone indicates significant volume loss with minimal new boney material.

### Immunohistochemistry and Histology

Identification of *bona fide* articular cartilage can be accomplished by studying ultrastructural and/or biochemical parameters. Articular cartilage forms a continuous layer of cartilage tissue possessing identifiable zones. The superficial-zone is characterized by chondrocytes having a flattened morphology and an extracellular network that stains poorly with toluidine blue, indicating relative absence of sulfated glycosaminoglycans (predominantly aggrecan). Chondrocytes in the mid- and deep-zones have a spherical appearance, and the matrix contains abundant sulfated proteoglycans, as evidenced by staining with toluidine blue.

Von Kossa staining shows a dense black staining of the mineralized tissue. This stain clearly depicts the existing and newly regenerated bone through the deposition of silver on the calcium salts. Typically, the counter stain is Safranin O, which stains the cartilage red-orange. New and existing bone can be easily distinguished morphologically in sections in this way. Safranin O/fast Green is able to distinguish more features than toluidine blue. Safranin O is a basic dye that stains the proteoglycans in the articular cartilage red-orange and the underlying subchondral bone only lightly. Fast Green is an acidic dye that stains the cytoplasm gray-green. This stain is not only able to clearly identify the existing and regenerated cartilage, but can also distinguish differences between the two regions, thereby indicating differences in the content of proteoglycans.

H&E stains bone a dark red and proteoglycan-rich cartilage only lightly.

Masson Trichrome distinguishes differences in reparative tissue. Cartilage and sulfated-glycosaminoglycan-rich reparative tissue is stained red, with the collagen of bone stained blue.

Histological evaluation can involve assessment of one or more of the following: glycosaminoglycan content in the repair cartilage; cartilage and chondrocyte morphology; and structural integrity and morphology at the defect interface. The morphology of repair cartilage can be identified by the type of cartilage formed: articular vs. fibrotic by glycosaminoglycan content, degree of cartilage deposition, organization of cells and collagen fibers.

The presence of collagen type II in cartilage tissue is an accepted phenotypic marker of differentiated chondrocytes. Standard gel electrophoresis, Western blot analysis, and/or immuno-histochemical staining can determine presence of collagen II. Staining for collagen types I and II is useful to determine the boundary between regenerated subchondral bone and reparative tissue. Generally, reparative tissue that is fibrous stains less intensely. Additionally, newly formed subchondral bone can be identified by collagen type II localization in small spicules of remnant cartilage.

A common scale used to assess repair of osteochondral defects has been developed by O'Driscoll, and a modification shown here:

| **Parameter** | **Points** |
|---|---|
| *Tissue Morphology* | |
| Mostly hyaline cartilage | 3 |
| Mostly fibrocartilage | 2 |
| Mostly non-cartilage | 1 |
| Non-cartilage only | 0 |

| *Matrix Staining (Safranin O)* | |
|---|---|
| Normal or nearly normal | 3 |
| Moderate | 2 |
| Slight | 1 |
| None | 0 |

| *Structural Integrity* | |
|---|---|
| Normal | 4 |
| Beginning of columnar organization | 3 |
| No organization | 2 |
| Cysts or disruptions | 1 |
| Severe disintegration | 0 |

| *Chondrocyte Clustering* | |
|---|---|
| No clusters | 2 |
| <25% of the cells | 1 |
| 25-100% of the cells | 0 |

| *Formation of Tidemark* | |
|---|---|
| Complete | 4 |
| 76-90% | 3 |
| 50-75% | 2 |
| 25-49% | 1 |
| <25% | 0 |

| *Subchondral Bone Formation* | |
|---|---|
| Good | 2 |
| Slight | 1 |
| No formation | 0 |

| *Architecture of Surface* | |
|---|---|
| Normal | 3 |
| Slight fibrillation or irregularity | 2 |
| Moderate fibrillation or irregularity | 1 |
| Severe fibrillation or disruption | 0 |

| *Filling of the Defect* | |
|---|---|
| 111-125% | 3 |
| 91-110% | 4 |
| 76-90 % | 3 |
| 51-75 % | 2 |
| 26-50 % | 1 |
| <25 % | 0 |

| *Lateral Integration* | |
|---|---|
| Bonded at both ends of graft | 2 |
| Bonded at one end/partially at both sides | 1 |
| Not bonded | 0 |

| *Basal Integration* | |
|---|---|
| 91-100% | 3 |
| 70-90 % | 2 |
| 50-70 % | 1 |
| <50 % | 0 |

| *Inflammation* | |
|---|---|
| No inflammation | 4 |
| Slight inflammation | 2 |
| Strong inflammation | 0 |
| | |
| **Maximum points possible** | **34** |

The empty defect showed essentially no new bone formation, with the defect size unchanged; however there was evidence of limited cartilage formation overlying the fibrotic tissue and penetrating down the walls of the defect. The most robust bone repair was seen in the defect filled with Cymetra Putty with more than 70% of the defect containing trabecular bone. In contrast, although the Cymetra/Fibrin combination appeared to be inhibitory to bone remodeling with the defect filled with original matrix material, the cartilage repair observed with this implant was superior to the empty defect and other implant materials.

Scoring of these implants for osteochondral repair, encompassing both bone and cartilage repair, is as follows:

| Implant | Total Score |
|---|---|
| Empty defect | 7 |
| Cymetra | 17 |
| Cymetra + Fibrin | 17 |
| Cymetra Putty | 18 |

All implant material scored significantly better than the no treatment control. However, it was noted that the Cymetra/Fibrin combination scored better on cartilage repair measures, and the Cymetra Putty scored better on trabecular bone repair. Nevertheless, compared to untreated defects, the combinations of acellular matrix implants were osteoconductive (that is, allowed for bone repair), and act as a scaffold for cartilage repair.

### Example 3. Remodelling of Periosteum in Porcine Segmental Defect Model

A mid-shaft segmental defect measuring two times the diameter of the bone (approximately 3 cm) was surgically created unilaterally in one femur of each of two pigs. The defect was thus a critical size defect which would not heal spontaneously. A metallic bone plate fixed to the bone with screws was applied across the defect, thereby fixing the osteomized bone in a correct anatomic position. A sheet of XenoDerm was reconstituted by soaking in saline for approximately ten minutes prior to surgical application. The XenoDerm sheet was wrapped around the cylindrical bone defect creating a tube. The sheet was overlapped on the proximal and distal ends of the bone on either side of gap by approximately 5mm and secured with sutures to the periosteum. Prior to closing of the XenoDerm tube, the tube defined by the XenoDerm sheet was filled with a 1:1 mixture of OSTEOSET® (calcium sulfate) pellets and cancellous autograft bone obtained from the proximal humerus of the recipient pig. After filling the tube with the graft materials, the XenoDerm sheet was closed along its length as a seam using sutures in a continuous pattern.

Radiographic analysis was done post-operatively, and at three and six week time points. Histological analysis was conducted at the conclusion of the six week study. Routine hematoxylin and eosin (H&E) staining was performed on the segmental defect sections.

The pigs resumed weight bearing on the operated limbs within 5 days of surgery and the wounds healed in a routine manner. The post-operative and three week radiographs showed that the defects remained stabilized in both pigs without fracture of the bone or breakage of the plates or screws. Each pig had one screw loosen by three weeks post surgery and several screws were loosened in one pig at six weeks. After six weeks, both pigs had a periosteal reaction (resulting in the formation of callus) over the cranial and lateral aspect of the femur encompassing the plate to varying degrees. Varying amounts of new bone were present in the defects of both pigs. The proximal and distal ends of the native femur exhibited proliferation of bone from the periosteal, cortical, and medullary surfaces. This bone extended into the defect as an initial phase of reestablishing the diaphyseal medullary canal.

Post-mortem radiographs (Fig. 2) show a considerable amount of new bone formed in the defect, resembling an early tubular structure which appears to penetrate within the margins of the implanted membrane. Although a solid tubular structure was not completely reconstructed at this early six week time point, there were struts of new bone formation bridging the defects.

The histological sections from the two pigs indicate that the acellular matrix (XenoDerm) functions as a biochemical and physical guide for new bone formation in a segmental defect by providing an environment for healing. The histological sections demonstrate new bone formation which penetrates within the three dimensional matrix of implanted matrices. Thus, the collagen bundles of the matrix are seen interlaced with newly formed bone indicating that new bone was actually formed within the matrix as well as adjacent to it. Some of the new bone within the matrix appeared from the histology to form through an initial cartilaginous phase.

This study indicates the ability of the acellular matrix to protect an underlying bone defect site and provide a protected environment for healing in a challenging segmental defect model. The grafted matrices remained at the defect site and there was abundant cellular activity within the matrices themselves. Indeed some new bone was formed within the matrices as well as along its margins. Thus, it appears that the implanted acellular dermal matrices (XenoDerm) remodeled to function in a manner essentially the same as normal periosteum in stimulating new bone formation adjacent to it, and also induced new bone formation within itself.

### Reference Example: Use of Acellular Matrices to Correct Congenital Myocardial Defects and Repair

### Damaged Cardiac Venticles

Two rat heterotopic heart graft models are tested. One is a model of an ischemic ventricular defect (the "ischemia model") and the other is a model of congenital left heart hyperplasia (the "hypoplastic left heart model"). In the ischemia model, the left main coronary artery is ligated, the ischemic area is excised, and the relevant segment of myocardium is replaced with a matrix having identical proportions to the excised segment. The manipulation preserves the overall ventricular shape and geometry. The hypoplastic left heart model involves no arterial ligation or excision but incision and patch expansion of the left ventricular wall as is needed to enlarge the overall size of the ventricular cavity. Moreover, in both these models, by appropriately manipulating the anastomotic connections of the donor heart [Ono et al. (1969) J. Thor. Cardiovasc. Surg. 57:225-229; Asfour et al. (1999) J. Heart and Lung Transplantation 18:927-936], it is possible to create either a functional (i.e., normal ventricular filling) or unloaded (ventricle bypassed) left ventricle. The matrix implanted in the ventricle is constructed in a two layered fashion with a 1 mm layer of Gore-Tex™ (polytetrafluoroethylene; PTFE), (W.L. Gore & Associates, Inc., Flagstaff, AZ) for strength and support and an internal layer of acellular matrix (e.g., AlloDerm®, XenoDerm, or acellular vascular matrix) to guide tissue regeneration. As an alterntative, two sheets of acellular matrix with particulate matrix between the sheets can be used.

Syngeneic male Lewis rats served as both cardiac donors and recipients in the heterotopic heart transplant model which has been extensively described [Ono et al., supra; Asfour et al., supra]. After systemic heparinization and cold cardioplegia of the donor, the donor heart is removed from the thorax with four separate ligatures, tying off the superior vena cava (SVC), inferior vena cava, and the right and left lung including the left SVC.

To create the hypoplastic left heart model, a 5mm incision is made in the left ventricle lateral to the left anterior descending artery. The ventricular cavity is then expanded by insertion of a 4mm x 4 mm x 2mm two layer construct (as described above). The complete construct is then secured in place with a running, locking 80 Nylon suture. The locking suture adequately achieves hemostasis and bleeding at this anastomis is unlikely to be a problem. Implantation of this graft by and end-to-side anastomosis of the donor aortic arch to the recipient infrarenal aorta and donor pulmonary artery to the recipient infrarenal inferior vena cava creates a fully unloaded left ventricle and the transplanted heart functions as an arterio-venous shunt. Oxygenated arterial blood passes through the recipient aorta to the donor aorta and coronary arteries, perfuses the myocardium, and is drained through the coronary sinus to the right atrium and ventricle to be ejected into the recipient inferior vena cava. A minor modification can create a volume loaded, fully functional left ventricle through the anastomosis of the donor pulomonary artery to the donor left atrium. The heart is then transplanted by an end-to-side anastomosis of the donor SVC to the recipient infrarenal inferior vena cava and an end-to-side anastomosis of the donor aortic arch to the recipient infrarenal aorta. Venous blood from the recipient inferior vena cava enters the donor SVC, passes through the right atrium and ventricle, and is ejected into the donor left atrium. After passing through the left atrium and ventricle it is ejected into the recipient aorta.

The ischemia model of the unloaded and fully loaded left ventricle hearts is created by a slight modification of this technique with ligation of the left anterior descending artery just distal to the first diagonal branch, full thickness excision of a 4mm x 4mm area of the left ventricular wall rendered ischemic, and implantation of the 4mm x 4mm x 2mm construct into the defect. This reconstruction preserves the overall geometry of the left ventricle. Control animals undergo an identical procedure except that no myocardium is excised and a ventricular patch is not implanted.

A simple ventriculostomy with immediate closure serves as control for the hypoplastic model and ligation of the left anterior descending artery without excision of the ischemic myocardium serves as a control for the ischemia model.

The four experimental groups are as follows: (1) Ischemic and loaded + matrix; (2) Ischemic and unloaded + matrix; (3) Hypoplastic and loaded + matrix; and (4) Hypoplastic and unloaded + matrix.

The four control groups are as follows: (1) Ischemic and loaded, no matrix; (2) Ischemic and unloaded, no matrix; (3) Hypoplastic and loaded, no matrix; and (4) Hypoplastic and unloaded, no matrix.

Alloderm, XenoDerm, and an equivalent acellular vascular matrix are tested in separate experiments.

At the completion of the surgical procedure, the animals are allowed to recover with free access to food and water. The animals in all groups are given 5-bromo-2'-deoxyuridine (BrdU) in their drinking water (0.8 mg/ml) for the duration of the experiment and are analyzed for myocardial regeneration at one month and two months post transplantation. At these time points, animals are sacrificed and the hearts fixed in distention with 10% phosphate buffered formalin, embedded in paraffin, and representative areas encompassing the implanted extracellular matrix sectioned into 5 micron coronal slices. A portion of these sections is stained with H&E and the morphology, cellularity, and organizational pattern of cellular ingrowth is compared to that of the surrounding heart. Since BrdU is a thymidine analog that is incorporated into the DNA during the S phase of the cell cycle, only cells that have divided can incorporate the nucleotide analog. By immunohistochemical evaluation utilizing both cardiomyocyte specific antibodies such as anti-myosin heavy chain monoclonal antibody (Sigma), and anti-troponin C mouse monoclonal antibody (Novocastra Laboratories Ltd.) as well as anti-BrdU specific antibodies, cardiac myocytes or myocyte precursors that have divided and differentiated into cardiac muscle can be identified. Vascularity of the neoventricular tissue is evaluated by counting capillary and arterial density after immunohistochemical staining of vascular endothelium with mouse anti-endothelial cell antibody (CD31; PECAM-1) (Dako Corp., Carpinteria, CA). Quantitative comparison of regeneration between the experimental and control groups is performed by counting the numbers of regenerating cardiac myocytes that have incorporated BrdU.

Myocardial function is assessed utilizing a bench top Langendorff preparation. After systemic heparinization the heterotopic heart will be isolated and perfused in a Langendorff apparatus with filtered Krebs-Henseleit buffer equilibrated with 5% carbon dioxide and 95% oxygen [Fremes et al. (1995) Annals. Thor. Surg. 59:1127-1133]. A latex balloon is passed into the left ventricle through the mitral valve and connected to a pressure transducer. The balloon size is then increased in 0.02 mL increments from 0.04 to 0.46 mL by the addition of saline solution while the systolic and diastolic pressures are recorded. The developed pressure at each volume reflects left ventricular function and is calculated as the difference between the systolic and diastolic pressure.

The regeneration potential of particulate acellular matrices delivered directly to an area of myocardial scar is investigated in a separate series of experiments. To study this phenomenon the donor heart is excised as described above and the left main coronary artery is ligated. The donor heart is then transplanted into the abdomen of a syngeneic recipient in order to create either a loaded or unloaded left ventricle as described above.

One month after infarct, at the completion of scar remodeling and matrix lysis by the inflammatory response, the heterotopic heart is temporarily arrested by cold cardioplegia and the area of the infarct is injected with the micronized form of AlloDerm (i.e., Cymetra) and XenoDerm respectively in two separate experimental groups. A control group undergoes the same manipulation except saline only is injected into the area of the scar.

The four experimental groups are as follows: (1) Ischemic and loaded + micronized AlloDerm; (2) Ischemic and unloaded + micronized AlloDerm; (3) Ischemic and loaded + micronized XenoDerm; and (4) Ischemic and unloaded + micronized XenoDerm.

The two control groups are as follows: (1) Ischemic and loaded + saline only; and (2) Ischemic and unloaded + saline only.

At the completion of the surgical procedure the animals are allowed to recover with free access to food and water. The animals in both experimental and control groups are given (BrdU) in their drinking water (see above) for the duration of the experiment and are analyzed at two weeks, one month, two months, and three month post transplantation by methods described above. Immunohistochemical staining for cardiomyocyte specific structural proteins and BrdU are used to identify cardiac myocyte or cardiac myocyte precursors that have divided and repopulated the area of the scar or extracellular matrix.

## Claims

1. A composition for use in treating a mammalian subject having a bone defect, comprising:
a hydrated gel, paste, or putty including a mixture of a particulate acellular dermal matrix and demineralized bone powder adapted to be placed in the bone defect of the mammalian subject.

2. The composition for use according to claim 1, wherein the mammalian subject is a human.

3. The composition for use according to claim 1, wherein the composition further comprises viable cells histocompatible with the mammalian subject.

4. The composition for use according to claim 1, wherein the cells are selected from the group consisting of epidermal cells, keratinocytes, endothelial cells, fibroblasts, embryonic stem cells of non-human origin, adult or embryonic mesenchymal stem cells of non-human origin, umbilical cord stem cells, prochondroblasts, chondroblasts, chondrocytes, pro-osteoblasts, osteocytes, osteoclasts, monocytes, pro-cardiomyoblasts, pericytes, cardiomyoblasts, carciomyocytes, gingival epithelial cells and periodontal ligament stem cells.

5. The composition for use according to claim 1, further comprising one or more agents selected from the group consisting of a cell growth factor, an angiogenic factor, a differentiation factor, a cytokine, a hormone and a chemokine.

6. The composition for use according to claim 5, wherein the one or more agents are in the composition placed in the bone defect of the mammalian subject.

7. The composition for use according to claim 6, wherein the composition comprises one or more expression vectors containing one or more nucleic acid sequences encoding the one or more agents, wherein each of the one or more nucleic acid sequences is operably linked to a transcriptional or a translational regulatory element.

8. The composition for use according to claim 7, wherein the one or more expression vectors are in one or more cells that are adapted to be administered to the mammalian subject.

9. The composition for use according to claim 8, wherein the one or more cells are in the composition.

10. The composition for use according to claim 1, wherein the composition is adapted to be placed within the bone defect.

11. The composition for use according to claim 1, further comprising a sheet of acellular tissue matrix adapted to cover the bone defect.

## Patentansprüche

1. Zusammensetzung zur Verwendung bei der Behandlung eines Säugetiers, das einen Knochendefekt hat, umfassend:
ein hydratisiertes Gel, Paste oder Masse umfassend eine Mischung aus einer partikelförmigen, azellulären, dermalen Matrix und demineralisiertem Knochenpulver, welche zum Einsetzen in den Knochendefekt des Säugetiers geeignet ist.

2. Zusammensetzung zur Verwendung nach Anspruch 1, wobei das Säugetier ein Mensch ist.

3. Zusammensetzung zur Verwendung nach Anspruch 1, wobei die Zusammensetzung ferner lebensfähige Zellen, die histokompatibel mit dem Säugetier sind, umfasst.

4. Zusammensetzung zur Verwendung nach Anspruch 1, wobei die Zellen ausgewählt sind aus einer Gruppe bestehend aus epidermalen Zellen, Keratinocyten, endothelialen Zellen, Fibroblasten, embryonalen Stammzellen nichtmenschlichen Ursprungs, adulten oder embryonalen mesenchymalen Stammzellen nichtmenschlichen Ursprungs, Nabelschnurstammzellen, Prochondroblasten, Chondroblasten, Chondrozyten, Prä-Osteoblasten, Osteozyten, Osteoklasten, Monozyten, Prokardiomyoblasten, Perizyten, Kardiomyoblasten, Kardiomyozyten, Zahnfleischepithelzellen und Periodontalligamentstammzellen.

5. Zusammensetzung zur Verwendung nach Anspruch 1, ferner umfassend ein oder mehrere Agenzien ausgewählt aus einer Gruppe bestehend aus Zellwachstumsfaktor, angiogenem Faktor, Differenzierungsfaktor, Zytokin, Hormon und Chemokin.

6. Zusammensetzung zur Verwendung nach Anspruch 5, wobei die ein oder mehreren Agenzien in der Zusammensetzung, die in den Knochendefekt des Säugetiers eingesetzt ist, enthalten sind.

7. Zusammensetzung zur Verwendung nach Anspruch 6, wobei die Zusammensetzung einen oder mehrere Expressionsvektoren umfasst, wobei die Expressionsvektoren eine oder mehrere Nukleinsäuresequenzen, die das eine oder die mehreren Agenzien codiert, umfassen, wobei jede der einen oder mehreren Nukleinsäuresequenzen mit einem regulatorischen Element für die Transkription oder Translation funktionsfähig verbunden ist.

8. Zusammensetzung zur Verwendung nach Anspruch 7, wobei die ein oder mehreren Expressionsvektoren in einer oder mehreren Zellen enthalten sind, die zur Verabreichung an das Säugetier geeignet sind.

9. Zusammensetzung zur Verwendung nach Anspruch 8, wobei die ein oder mehreren Zellen in der Zusammensetzung enthalten sind.

10. Zusammensetzung zur Verwendung nach Anspruch 1, wobei die Zusammensetzung dazu geeignet ist, in den Knochendefekt platziert zu werden.

11. Zusammensetzung zur Verwendung nach Anspruch 1, ferner umfassend eine Lage von azellulärer Gewebematrix, die dazu geeignet ist, den Knochendefekt abzudecken.

## Revendications

1. Composition destinée à un une utilisation dans le traitement d'un sujet mammifère présentant un défaut osseux, comprenant : un gel, une pâte ou un mastic hydratés contenant un mélange d'une matrice dermique acellulaire particulaire et d'une poudre d'os déminéralisée conçu pour être mis en place dans le défaut osseux du sujet mammifère.

2. Composition destinée à une utilisation selon la revendication 1, le sujet mammifère étant un humain.

3. Composition destinée à une utilisation selon la revendication 1, la composition comprenant en outre des cellules viables histocompatibles avec le sujet mammifère.

4. Composition destinée à une utilisation selon la revendication 1, où les cellules sont choisies dans le groupe constitué par les cellules épidermiques, les kératinocytes, les cellules endothéliales, les fibroblastes, les cellules souches embryonnaires d'origine non humaine, les cellules souches mésenchymateuses adultes ou embryonnaires d'origine non humaine, les cellules souches du cordon ombilical, les prochondroblastes, les chondroblastes, les chondrocytes, les pro-ostéoblastes, les ostéocytes, les ostéoclastes, les monocytes, les pro-cardiomyoblastes, les péricytes, les cardiomyoblastes, les cardiomyocytes, les cellules épithéliales gingivales et les cellules souches des ligaments parodontiques.

5. Composition destinée à une utilisation selon la revendication 1, comprenant en outre un ou plusieurs agents choisis dans le groupe constitué par un facteur de croissance cellulaire, un facteur angiogénique, un facteur de différenciation, une cytokine, une hormone et une chimiokine.

6. Composition destinée à une utilisation selon la revendication 5, dans laquelle le ou les agents sont dans la composition mise en place dans le défaut osseux du sujet mammifère.

7. Composition destinée à une utilisation selon la revendication 6, la composition comprenant un ou plusieurs vecteurs d'expression contenant une ou plusieurs séquences d'acides nucléiques codant pour le ou les agents, chacune de la ou des séquences d'acides nucléiques étant fonctionnellement liée à un élément régulateur de transcription ou de traduction.

8. Composition destinée à une utilisation selon la revendication 7, dans laquelle le ou les vecteurs d'expression se trouvent dans une ou plusieurs cellules conçues pour être administrées au sujet mammifère.

9. Composition destinée à une utilisation selon la revendication 8, la ou les cellules se trouvant dans la composition.

10. Composition destinée à une utilisation selon la revendication 1, la composition étant conçue pour être mise en place dans le défaut osseux.

11. Composition destinée à une utilisation selon la revendication 1, comprenant en outre un feuillet d'une matrice tissulaire acellulaire conçue pour recouvrir le défaut osseux.
